# EUROPÄISCHE PATENTANMELDUNG

(11) **EP 3 986 088 A1**
(43) Veröffentlichungstag der Anmeldung: **20.04.2022**
(21) Anmeldenummer: 21201613.3
(22) Anmeldetag: 08.10.2021
(51) Int. Cl.: H05B 3/34, A61F 7/08, H05B 1/02

(54) **WÄRMEKISSEN FÜR DEN MENSCHLICHEN KÖRPER ZUR LINDERUNG VON KÖRPERLICHEN BESCHWERDEN**

(30) Priorität: 14.10.2020 DE 102020127028
(71) Anmelder: cebeha2 GmbH, 81373 München (DE)
(72) Erfinder: Hader, Carina, 81373 München (DE); Scheile, Felix, 80801 München (DE)
(74) Vertreter: Canzler & Bergmeier Patentanwälte Partnerschaft mbB

(57) **Zusammenfassung**

Die Erfindung betrifft ein Wärmekissen (1) für den menschlichen Körper zur Linderung von körperlichen Beschwerden, insbesondere Dysmenorrhoe, mit wenigstens einer Energiespeichereinrichtung (2) zum Speichern von elektrischer Energie und einem Heizelement (4) zum Erwärmen des Wärmekissens (1) mittels der elektrischen Energie. Das Wärmekissen (1) weist wenigstens eine Leiterbahn (6) zum Leiten von Signalen und/oder der elektrischen Energie auf, wobei das Heizelement (4), die Energiespeichereinrichtung (2) und die Leiterbahn (6), insbesondere vollständig, von einer Hülle (14) ummantelt sind.

## Beschreibung

Die vorliegende Erfindung betrifft ein Wärmekissen für den menschlichen Körper zur Linderung von körperlichen Beschwerden, insbesondere Dysmenorrhoe, mit wenigstens einem Heizelement zum Erwärmen des Wärmekissens und einer Energiespeichereinrichtung zum Speichern von elektrischer Energie.

Aus der EP 0 042 448 A1 ist ein Wärmekissen mit einem flexiblen Heizelement bekannt, welcher in einer Isolationsmasse eingebettet und flächenförmig in einer Hülle aus einem Textilgewebe angeordnet ist. Das flexible Heizelement wird über Anschlussleitungen mit elektrischer Energie versorgt. Aus welcher Energiequelle diese elektrische Energie stammt, wird nicht erwähnt. Nachteilig daran ist, dass die Energie über Anschlussleitungen in das Wärmekissen eingebracht wird und somit eine mobile Nutzung des Wärmekissens wenig komfortabel ist. Zudem besteht durch die seitlich abstehenden Anschlussleitungen erhöhte Verletzungsgefahr.

Aufgabe der vorliegenden Erfindung ist es, die aus dem Stand der Technik bekannten Nachteile zu beseitigen. Aufgabe ist es insbesondere, ein Wärmekissen für den menschlichen Körper zur Linderung von körperlichen Beschwerden zu schaffen, welches die mobile Nutzung vereinfacht, den Wärmeeintrag in den Körper verbessert und die Sicherheit erhöht.

Die Aufgabe wird gelöst durch ein Wärmekissen für den menschlichen Körper zur Linderung von körperlichen Beschwerden mit den Merkmalen des unabhängigen Patentanspruchs.

Vorgeschlagen wird ein Wärmekissen für den menschlichen Körper zur Linderung von körperlichen Beschwerden, insbesondere Dysmenorrhoe, mit wenigstens einer Energiespeichereinrichtung zum Speichern von elektrischer Energie und einem Heizelement zum Erwärmen des Wärmekissens mittels der elektrischen Energie.

Als Energiespeichereinrichtung ist eine Einrichtung zu verstehen, welche aktuell verfügbare elektrische Energie speichert und für eine spätere Nutzung bereitstellt. Für die Speicherung der elektrischen Energie wird diese häufig in eine chemische Energie umgewandelt. Als Heizelement ist ein Element zu verstehen, welches die elektrische Energie in Wärme umwandelt. Das Heizelement kann beispielsweise einen elektrischen Widerstandsdraht aufweisen, der sich aufgrund des hohen elektrischen Widerstands bei Bestromung aufwärmt und die Wärme freisetzt.

Erfindungsgemäß weist das Wärmekissen wenigstens eine Leiterbahn zum Leiten von Signalen und/oder der elektrischen Energie auf, wobei das Heizelement, die Energiespeichereinrichtung und die Leiterbahn, insbesondere vollständig, von einer Hülle ummantelt sind. Die elektrische Energie wird dabei beispielsweise von der Energieeinrichtung zum Heizelement geleitet. Zusätzlich oder alternativ können auch Signale, insbesondere elektrische Signale, mittels der Leiterbahn(en) geleitet werden. Die Signale werden in Form von elektrischen Größen, wie beispielsweise der Stromstärke, der Spannung und/oder des elektrischen Widerstands, zum Austausch von Informationen über die Leiterbahn geleitet. So können zusätzlich zur elektrischen Energie auch Signale über die Leiterbahn geleitet und somit Informationen, wie beispielsweise zum Funktionszustand des Heizelements und/oder der Energiespeichereinrichtung, weitergegeben werden. Durch die, insbesondere vollständige, Ummantelung mit der Hülle kann zudem gewährleistet werden, dass der Anwender des Wärmekissens nicht mit der elektrischen Energie in Berührung kommt. So sind vorteilhafterweise alle elektronischen Bauteile des Wärmekissens von der Hülle ummantelt. Des Weiteren kommt durch die, insbesondere vollständige, Ummantelung mit der Hülle lediglich die Hülle mit dem Körper in Berührung. Dadurch muss nur die Hülle bei Verschmutzung gereinigt werden. Ist das Heizelement, die Energiespeichereinrichtung und die Leiterbahn vollständig von der Hülle ummantelt, kann das Eindringen von Wasser und/oder Fremdstoffen verhindert werden. Dadurch kann die Sicherheit beim Betreiben des Wärmekissens verbessert werden. Vorteilhafterweise ist die Hülle aus einem flexiblen und nichtleitenden Material, insbesondere aus Kunststoff, vorzugsweise aus Silikon und/oder TPE, gefertigt. Wird die Hülle aus Kunststoff, insbesondere Silikon und/oder TPE, gefertigt, so kann diese kostengünstig um die elektronischen Bauteile umspritzt oder umgossen werden. Darüber hinaus ist eine aus Silikon und/oder TPE gefertigte Hülle sehr flexibel und kann sich so an den Körper anpassen.

Vorteilhaft ist es, wenn das Wärmekissen wenigstens eine Leiterplatte aufweist, wobei die wenigstens eine Leiterbahn als Versorgungslage für die elektrische Energie und/oder als Signallage für die Signale an der Leiterplatte angeordnet ist. Als Leiterplatte ist hierbei ein im Wesentlichen plattenförmiges Element zu verstehen, welches elektronische Bauteile aufnimmt und diese elektrisch verbindet. So kann zusätzlich zur elektrischen Verbindung des Heizelements und/oder der Energiespeichereinrichtung eine mechanische Befestigung an der Leiterplatte erfolgen und somit deren Position innerhalb der Hülle des Wärmekissens festgelegt sein. Die Leiterplatte kann wenigstens eine Lage, vorteilhafterweise mehrere Lagen, aufweisen. Weist die Leiterplatte mehrere miteinander verbundene Lagen auf, so kann jede der Lagen eine unterschiedliche Funktion übernehmen. Die Lagen sind in eine Lagenaufbaurichtung aneinander angereiht und voneinander elektrisch isoliert. So kann die Versorgungslage als Leiterbahn zum Leiten der elektrischen Energie ausgebildet sein. Die Signallage kann als Leiterbahn zum Leiten der Signale ausgebildet sein. Zudem kann die Signallage und/oder die Versorgungslage in mehrere Lagen aufgeteilt sein. Dadurch kann die Leiterplatte das Leiten der elektrischen Energie und/oder der Signale und deren Trennung voneinander gewährleisten.

Auch ist es von Vorteil, wenn das Heizelement als Heizlage an der Leiterplatte angeordnet ist und/oder unmittelbar an der Hülle anliegt. Ist das Heizelement als Heizlage an der Leiterplatte angeordnet, so ist dieses als eine Lage der mehrlagigen Leiterplatte ausgebildet und/oder zumindest teilweise mit wenigstens einer der Lagen mechanisch und/oder elektrisch verbunden. So kann die mehrlagige Leiterplatte zum Leiten der elektrischen Energie und/oder der Signale das Wärmekissen zusätzlich erwärmen.

Ebenso ist es vorstellbar, dass das Heizelement als Heizlage an einer zusätzlichen Leiterplatte angeordnet ist. Diese zusätzliche Leiterplatte kann an die Leiterplatte nachträglich angebunden werden. Zusätzlich oder alternativ ist das Heizelement in Lagenaufbaurichtung unmittelbar an der Hülle angeordnet. Das Heizelement gibt somit die Wärme unmittelbar flächig auf die Hülle ab. Dadurch ist ein möglichst energiesparendes Aufwärmen des Wärmekissens gewährleistet.

Vorteile bringt es zudem mit sich, wenn das Wärmekissen eine Ladeeinrichtung zum Laden der Energiespeichereinrichtung aufweist. Bei zunehmender Nutzungsdauer des Wärmekissens wird die Energiespeichereinrichtung immer mehr entladen. Um der Energiespeichereinrichtung erneut elektrische Energie zuzuführen, ist diese mit der Ladeeinrichtung verbunden. Dadurch kann die Energiespeichereinrichtung geladen und somit das Wärmekissen wiederverwendet werden.

Auch ist es vorteilhaft, wenn die Ladeeinrichtung eine Induktionsspule aufweist, wobei die Ladeeinrichtung, insbesondere vollständig, von der Hülle ummantelt ist. So kann mittels elektromagnetischer Induktion, welche auf die Induktionsspule wirkt, die Ladeeinrichtung betrieben und so die Energiespeichereinrichtung geladen werden. Dadurch kann die Energiespeichereinrichtung des Wärmekissens geladen werden, ohne die Hülle mit einem elektrischen Leiter durchdringen zu müssen. Ist die Ladeeinrichtung zudem insbesondere vollständig von der Hülle ummantelt, kann das Eindringen von Wasser und/oder Fremdstoffen verhindert werden. Dadurch kann die Sicherheit beim Betreiben des Wärmekissens weiter verbessert werden.

Vorteilhaft ist es zudem, wenn das Wärmekissen ein Isolationselement zur thermischen Isolation der Ladeeinrichtung, der Energiespeichereinrichtung und/oder der Leiterplatte zur Hülle und/oder zum Heizelement aufweist. Das Wärmekissen liegt meist flächig und einseitig auf dem Körper auf. Der Bereich der Hülle, der am Körper anliegt, kann dabei als Anlagefläche bezeichnet werden. Um die Wärme des Heizelements zumindest zum Großteil in Richtung des Körpers und nicht an die Umgebung abzuleiten, ist das Heizelement in Lagenaufbaurichtung vorzugsweise näher an der Anlagefläche angeordnet, als das Isolationselement. Das Isolationselement kann dabei, insbesondere unmittelbar, in Lagenaufbaurichtung am Heizelement angeordnet sein. So wird die Ladeeinrichtung, die Energiespeichereinrichtung und/oder die Leiterplatte thermisch vom Heizelement isoliert. Dadurch kann der Wärmeübergang vom Heizelement in die Ladeeinrichtung, die Energiespeichereinrichtung und/oder die Leiterplatte minimiert werden. Zusätzlich oder alternativ kann das Isolationselement ebenso, insbesondere unmittelbar, in Lagenaufbaurichtung an der Hülle angeordnet sein. Dadurch wird die Ladeeinrichtung, die Energiespeichereinrichtung und/oder die Leiterplatte von der Hülle thermisch isoliert. Ist sowohl das Heizelement, als auch das Isolationselement unmittelbar an der Hülle angeordnet, so sind diese in Lagenaufbaurichtung maximal voneinander beabstandet. Das Heizelement ist hierbei an der Anlagefläche und das Isolationselement an der in Lagenaufbaurichtung gegenüberliegenden Fläche der Hülle angeordnet. Dadurch kann die Leiterplatte möglichst kompakt gestaltet und zusätzlich mittels des Isolationselements von der Hülle isoliert werden.

Ebenso bringt es Vorteile mit sich, wenn die Energiespeichereinrichtung eine Vielzahl an Akkumulatoren aufweist und/oder die Energiespeichereinrichtung zumindest teilweise flexibel ausgestaltet ist. Die Akkumulatoren können hierbei die elektrische Energie mittels einer elektrochemischen Reaktion speichern und mittels der Ladeeinrichtung aufgeladen und somit wiederverwendet werden. Um hierbei die notwendige elektrische Energie speichern zu können, kann die Energiespeichereinrichtung eine Vielzahl an miteinander in Wirkverbindung stehenden Akkumulatoren aufweisen. Zudem kann es für eine flexiblere Ausgestaltung des Wärmekissens von Vorteil sein, wenn die Energiespeichereinrichtung in eine Vielzahl an kleineren Akkumulatoren aufgeteilt ist. Ist die Energiespeichereinrichtung in eine Vielzahl an Akkumulatoren aufgeteilt, so sind die Zwischenräume, welche sich zwischen den einzelnen Akkumulatoren bilden, biegbar. Die Akkumulatoren können dabei nicht oder nur schlecht biegbar ausgestaltet sein. Zusätzlich oder alternativ kann die Energiespeichereinrichtung zumindest teilweise flexibel ausgestaltet sein. Hierfür kann die Energiespeichereinrichtung wenigstens einen Akkumulator aufweisen, welcher aus einem flexiblen Material besteht und somit biegbar ausgestaltet ist. Durch eine derartige flexible Ausgestaltung der Energiespeichereinrichtung kann sich das Wärmekissen flexibler an den Körper anlegen.

Des Weiteren ist es vorteilhaft, wenn die Leiterplatte wenigstens eine Aussparung aufweist und/oder teilweise aus einem flexiblen Material besteht. Als Aussparung ist hierbei ein Durchbruch zu verstehen, welcher die Leiterplatte in Lagenaufbaurichtung durchbricht. Aufgrund der durch die Aussparung entnommene Steifigkeit der Leiterplatte kann das Falten zumindest im Bereich der Aussparung vereinfacht werden. Zusätzlich oder alternativ kann die Leiterplatte zumindest teilweise aus dem flexiblen Material gestaltet sein. Leiterplatten, die vollständig aus dem flexiblen Material bestehen werden auch flexible Leiterplatten genannt. Leiterplatten, die nur teilweise aus dem flexiblen Material bestehen werden auch Starr-Flex-Leiterplatten genannt. Als flexibles Material für die Leiterplatte ist hierbei ein Material zu verstehen, welches elastisch biegbar ist. Derartige Leiterplatten werden häufig aus dünnen Folien, insbesondere aus Polyamid, PET oder PEN, gebildet. Aufgrund der zumindest teilweise Ausgestaltung der Leiterplatte als flexible Leiterplatte oder als Starr-Flex-Leiterplatte kann das Falten zumindest im Bereich des flexiblen Materials weiter vereinfacht werden. Zudem ist es vorteilhaft, wenn die Aussparung der Leiterplatte mit einem flexiblen, nichtleitenden Material, insbesondere dem Material der Hülle, gefüllt ist. So kann die Leiterplatte beim Falten vor einem Bruch geschützt werden.

Auch ist es vorteilhaft, wenn die Leiterplatte, die Hülle, das Heizelement, die Energiespeichereinrichtung, die Ladeeinrichtung und/oder die Aussparung zu einer Faltebene symmetrisch zum Falten des Wärmekissens von einer Betriebsstellung in eine Sicherungsstellung und zurück ausgebildet ist. Als Sicherungsstellung ist die Stellung zu verstehen, bei der das Wärmekissen im zusammengefalteten Zustand nicht verwendet wird. Als Betriebsstellung ist die Stellung zu verstehen, bei der das Wärmekissen im aufgefalteten Zustand am Körper angelegt werden kann. Um das gleichmäßige Falten des Wärmekissens zu gewährleisten, ist die Leiterplatte, die Hülle, das Heizelement, die Energiespeichereinrichtung, die Ladeeinrichtung und/oder die Aussparung zur Faltebene symmetrisch. Dadurch kann das Wärmekissen im Sicherungszustand möglichst kompakt gestaltet und so die mobile Nutzung vereinfacht werden. Zudem kann es zum Falten des Wärmekissens von Vorteil sein, wenn die Energiespeichereinrichtung in eine Vielzahl an Akkumulatoren aufgeteilt ist. So ist der Zwischenraum der Akkumulatoren flexibler gestaltet, woraus sich eine bessere Faltbarkeit des Wärmekissens ergibt. Darüber hinaus ist es vorstellbar, dass der Zwischenraum zwischen den Akkumulatoren mit dem Material der Hülle gefüllt ist.

Vorteilhaft ist es zudem, wenn das Wärmekissen wenigstens zwei Halteelemente zum Halten des Wärmekissens in der Sicherungsstellung aufweist, wobei die Halteelemente vorzugsweise symmetrisch zur Faltebene angeordnet sind. Dadurch kann ein ungewolltes Falten des Wärmekissens von der Sicherungsstellung in die Betriebsstellung verhindert werden. Die Halteelemente können beispielsweise Magnete und/oder Klettverschlüsse sein.

Des Weiteren ist es vorteilhaft, wenn das Wärmekissen eine Schalteinrichtung zum Schalten des Wärmekissens aufweist, wobei die Schalteinrichtung vorzugsweise wenigstens einen Reedschalter und/oder einen Magnetkontakt aufweist. Die Schalteinrichtung stellt dabei beim Schalten den Kontakt zwischen Energiespeichereinrichtung und Heizelement je nach Stellung her oder ab. Dadurch kann das Wärmekissen ein und/oder ausgeschaltet werden. Als Reedschalter ist hierbei ein Schalter zu verstehen, der durch ein Magnetfeld geschaltet wird. Reedschalter sind häufig in einem Glasröhrchen eingeschmolzene Kontaktzungen die auf das Magnetfeld reagieren. Nähert oder entfernt sich das Magnetfeld, so werden die Kontaktzungen durch das Magnetfeld zusammengeführt oder voneinander entfernt. Hierfür kann die Schalteinrichtung zusätzlich zu dem Reedschalter einen Magnetkontakt aufweisen. Der Magnetkontakt stellt dabei das Magnetfeld zum Schalten des Reedschalters her. Das Magnetfeld kann jedoch zusätzlich oder alternativ vom Halteelement, insbesondere vom Magneten das Halteelement, stammen. Zudem ist es von Vorteil, wenn der Reedschalter und das Magnetfelderzeugende Mittel, insbesondere der Magnetkontakt und/oder das Halteelement, symmetrisch zur Faltebene zueinander angeordnet sind. So kann gewährleistet werden, dass beim Falten des Wärmekissens von der Sicherungsstellung in die Betriebsstellung und zurück das Magnetfeld des Magnetkontakts und/oder des Halteelements am Reedschalter verändert wird.

Auch ist es vorteilhaft, wenn die Schalteinrichtung, insbesondere der Reedschalter und/oder der Magnetkontakt, derart eingerichtet ist, dass das Heizelement in der Sicherungsstellung von der Energiespeichereinrichtung entkoppelt ist. In der Sicherungsstellung ist das Wärmekissen zusammengefaltet. Handelt es sich bei der Schalteinrichtung um einen Reedschalter und einen Magnetkontakt, so wirkt das Magnetfeld des Magnetkontakts in der Sicherungsstellung auf den Reedschalter ein. Der Reedschalter ist somit derart gestaltet, dass er unter Einwirkung des Magnetfelds den Kontakt des Heizelements zur Energiespeichereinrichtung unterbricht. Somit kann der Reedschalter derart gestaltet sein, dass sich die Kontaktzungen des Reedschalters unter Einwirkung des Magnetfelds voneinander entfernen. Dadurch kann gewährleistet werden, dass in der Sicherungsstellung das Heizelement keine Wärme abgibt.

Vorteilhaft ist es zudem, wenn die Schalteinrichtung, insbesondere der Reedschalter und/oder der Magnetkontakt, derart eingerichtet ist, dass das Heizelement in der Betriebsstellung mit der Energiespeichereinrichtung verbunden ist. In der Betriebsstellung ist das Wärmekissen auseinandergefaltet. Handelt es sich bei der Schalteinrichtung um einen Reedschalter und einen Magnetkontakt, so wirkt das Magnetfeld des Magnetkontakts in der Betriebsstellung nicht auf den Reedschalter ein, da diese maximal voneinander beabstandet sind. Der Reedschalter ist somit derart gestaltet, dass er ohne vorhandenes Magnetfeld den Kontakt des Heizelements zur Energiespeichereinrichtung herstellt. Dadurch kann das Heizelement nach dem Aufklappen des Wärmekissens vom Sicherungszustand in den Betriebszustand Wärme abgeben.

Auch ist es vorteilhaft, wenn das Wärmekissen eine Steuereinrichtung zum Steuern und/oder Regeln der Energiespeichereinrichtung, des Heizelements, der Ladeeinrichtung und/oder der Schalteinrichtung aufweist, wobei die Steuereinrichtung vorzugsweise mit der Signallage der Leiterplatte zur Verarbeitung der Signale verbunden ist. Dadurch kann der Funktionszustand des Energiespeichers, des Heizelements, der Ladeeinrichtung und/oder der Schalteinrichtung geprüft, gesteuert und/oder geregelt werden. Die Steuereinrichtung kann hierbei vorzugsweise die Referenzspannung, die Ladeabschaltung und/oder die Unterspannungsabschaltung prüfen, steuern und/oder regeln. Bei der Ladeabschaltung kann beispielsweise bei vollständiger Ladung der Energiespeichereinrichtung die Ladeeinrichtung von der Energiespeichereinrichtung getrennt werden. So kann ein Überladen der Ladeeinrichtung vermieden werden. Bei der Unterspannungsabschaltung kann beispielsweise die Energiespeichereinrichtung von dem Heizelement getrennt werden, falls diese zu wenig elektrische Energie enthält. So kann eine Unterschreitung einer vorgegebenen Spannung der Energiespeichereinrichtung vermieden werden. Zusätzlich oder alternativ kann die Steuereinrichtung einen Temperatursensor zum Messen, Steuern und/oder Regeln der Temperatur des Wärmekissens aufweisen. Mittels des Temperatursensors kann die Temperatur des Wärmekissens, insbesondere des Heizelements, gemessen werden. Weicht die vom Temperatursensor gemessene Temperatur von einem vorab eingestellten Soll-Temperaturbereich ab, so kann mit Hilfe der Steuereinrichtung die Temperatur geregelt werden. So kann das Wärmekissen stets in einem Soll-Temperaturbereich betrieben werden.

Vorteilhaft ist es zudem, wenn die Energiespeichereinrichtung, das Heizelement, die Steuereinrichtung, die Schalteinrichtung und/oder die Ladeeinrichtung elektrisch leitend mit der wenigstens einen Leiterbahn, vorzugsweise mit der Leiterplatte, insbesondere der Signallage und/oder der Versorgungslage der Leiterplatte, verbunden ist. So kann der elektrische Kontakt zwischen den Bauteilen gewährleistet werden.

Des Weiteren ist es vorteilhaft, wenn der Lagenaufbau des Wärmekissens derart ausgebildet ist, dass sich das Heizelement in der Sicherungsstellung näher an der Innenseite und die Ladeeinrichtung näher an der Außenseite des Wärmekissens befindet. Weist die Ladeeinrichtung eine Induktionsspule auf, so wird diese über elektromagnetischer Induktion beaufschlagt. Vorteilhafterweise wird die Ladeeinrichtung in der Sicherungsstellung betrieben. Befindet sich die Ladeeinrichtung in der Sicherungsstellung näher an der Außenseite des Wärmekissens, wird die elektromagnetische Induktion durch den Lagenaufbau weniger beeinflusst. Dadurch kann das Betreiben der Ladeeinrichtung vereinfacht werden.

Weitere Vorteile der Erfindung sind in den nachfolgenden Ausführungsbeispielen beschrieben. Es zeigt:
- **Figur 1**: eine schematische Draufsicht des Inneren eines Wärmekissens in einer Betriebsstellung gemäß einem Ausführungsbeispiel,
- **Figur 2a**: eine schematische Draufsicht eines Wärmekissens gemäß einem alternativen Ausführungsbeispiel,
- **Figur 2b**: eine schematische Untersicht eines Wärmekissens gemäß einem Ausführungsbeispiel ähnlich der Figur 2a,
- **Figur 3**: ein schematischer Lagenaufbau eines Wärmekissens gemäß einem weiteren Ausführungsbeispiel,
- **Figur 4a**: eine schematische räumliche Darstellung des Wärmekissens in einer Betriebsstellung gemäß einem weiteren alternativen Ausführungsbeispiel, und
- **Figur 4b**: eine schematische räumliche Darstellung des Wärmekissens in einer Sicherungsstellung gemäß einem Ausführungsbeispiel ähnlich der Figur 4a.

Bei der nachfolgenden Beschreibung der Figuren werden für in den verschiedenen Figuren jeweils identische und/oder zumindest vergleichbare Merkmale gleiche Bezugszeichen verwendet. Die einzelnen Merkmale, deren Ausgestaltung und/oder Wirkweise werden meist nur bei ihrer ersten Erwähnung ausführlich erläutert. Werden einzelne Merkmale nicht nochmals detailliert erläutert, so entspricht deren Ausgestaltung und/oder Wirkweise der Ausgestaltung und Wirkweise der bereits beschriebenen gleichwirkenden oder gleichnamigen Merkmale.

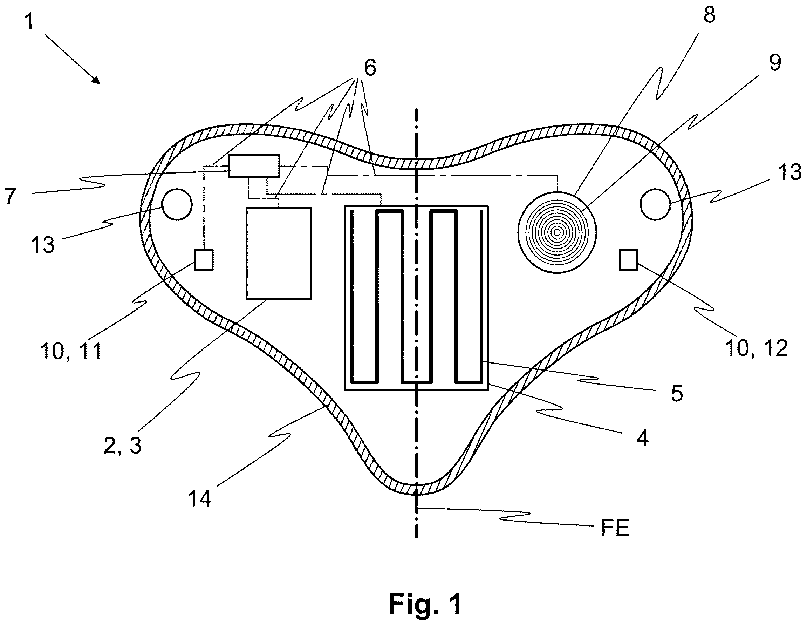

Figur 1 zeigt eine schematische Draufsicht des Inneren eines Wärmekissens 1 in einer Betriebsstellung gemäß einem Ausführungsbeispiel. Das Wärmekissen 1 weist eine Energiespeichereinrichtung 2 mit wenigstens einem Ackumulator 3 zum Speichern von elektrischer Energie auf. Im gezeigten Ausführungsbeispiel weist die Energiespeichereinrichtung 2 einen Akkumulator 3 auf. Zur Erwärmung weist das Wärmekissen 1 ein Heizelement 4 auf. Hierfür wird die elektrische Energie durch einen elektrischen Widerstandsdraht 5 des Heizelements 4 geleitet. Aufgrund des hohen elektrischen Widerstands des Widerstandsdrahtes 5 wird Wärme freigesetzt und so das Wärmekissen 1 erwärmt.

Um Signale und/oder die elektrische Energie von der Energiespeichereinrichtung 2 zum Heizelement 4 zu leiten, weist das Wärmekissen 1 wenigstens eine Leiterbahn 6 auf. Die Signale und/oder die elektrische Energie können hierbei direkt mittels einer Leiterbahn 6 von der Energiespeichereinrichtung 2 zum Heizelement 4 oder wie hier dargestellt über eine dazwischengeschaltete Steuereinrichtung 7 geleitet werden. Die Steuereinrichtung 7 ist mit einer Vielzahl an elektronischen Bauteilen versehen, welche eine Vielzahl an Funktionen im Wärmekissen 1 prüfen, steuern und/oder regeln können. So kann die Steuereinrichtung 7 die elektrische Verbindung zwischen der Energiespeichereinrichtung 2 und dem Heizelement 4 mittels der Leiterbahnen 6 herstellen. Wurde die gespeicherte Energie der Energiespeichereinrichtung 2 zu einem vorher definierten Teil bereits durch das Heizelement 4 in Wärme umgewandelt, so kann die Steuereinrichtung 7 die elektrische Verbindung zwischen der Energiespeichereinrichtung 2 und dem Heizelement 4 trennen. Durch diese sogenannte Unterspannungsabschaltung kann verhindert werden, dass der Energiespeichereinrichtung 2 zu viel elektrische Energie entnommen wird.

Das Wärmekissen 1 weist eine Ladeeinrichtung 8 zum Laden der Energiespeichereinrichtung 2 auf. Mittels der Ladeeinrichtung 8 kann die zumindest teilweise entladene Energiespeichereinrichtung 2 mit elektrischer Energie versorgt werden. Im gezeigten Ausführungsbeispiel weist die Ladeeinrichtung 8 eine Induktionsspule 9 auf. Dadurch kann mittels elektromagnetischer Induktion, welche auf die Induktionsspule 9 von außen einwirkt, die Ladeeinrichtung 8 betrieben und so die Energiespeichereinrichtung 2 mit dem Akkumulator 3 geladen werden. Die Ladeeinrichtung 8 ist dabei über eine der Leiterbahnen 6 über der Regeleinrichtung 7 mit der Energiespeichereinrichtung 2 verbunden. Die Regeleinrichtung 7 kann dabei insbesondere dann die Verbindung von Ladeeinrichtung 8 zur Energiespeichereinrichtung 2 trennen, wenn die Energiespeichereinrichtung 2 vollgeladen ist. Dies wird als Ladeabschaltung bezeichnet und verhindert ein Überladen der Energiespeichereinrichtung 2.

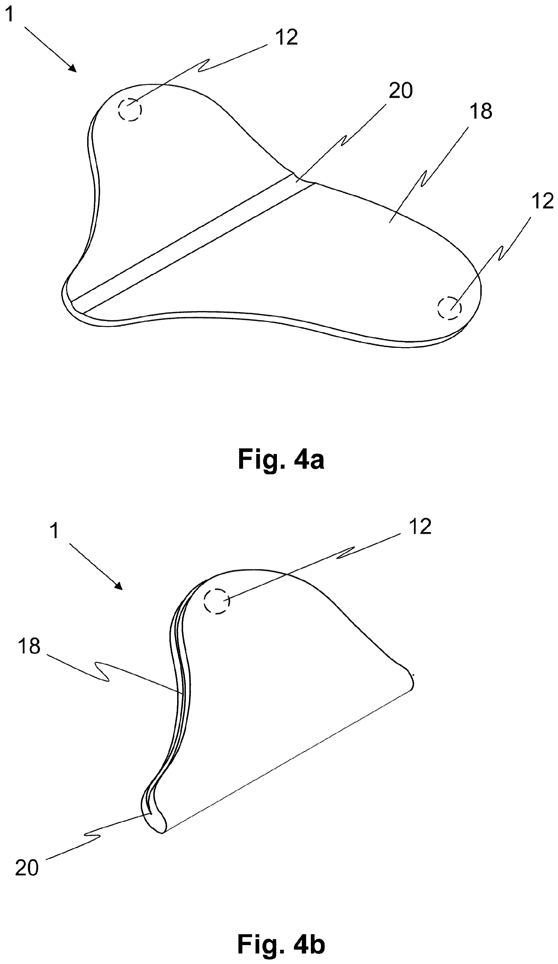

Das Wärmekissen 1 weist eine Schalteinrichtung 10 auf. Die Schalteinrichtung 10 schaltet das Wärmekissen 1, insbesondere die elektrische Verbindung von der Energiespeichereinrichtung 2 zum Heizelement 4. Im gezeigten Ausführungsbeispiel ist die Schalteinrichtung 10 mittels der Steuereinrichtung 7 über Leiterbahnen 6 mit der Energiespeichereinrichtung 2 und dem Heizelement 4 verbunden. Die Schalteinrichtung 10 weist einen Reedschalter 11 und einen Magnetkontakt 12 auf, wobei der Reedschalter 11 mit der Steuereinrichtung 7 über die Leiterbahn 6 verbunden ist. Der Reedschalter 11 und der Magnetkontakt 12 sind hierbei in ihrer Position symmetrisch zu einer Faltebene FE angeordnet. Wird das Wärmekissen 1 entlang der Faltebene FE gefaltet, so wird das Wärmekissen 1 von der hier dargestellten Betriebsstellung in eine Sicherungsstellung (siehe Figur 4b) gebracht. In der Sicherungsstellung liegen der Reedschalter 11 und der Magnetkontakt 12, aufgrund ihrer zur Faltebene FE symmetrischen Anordnung, derart aneinander, dass das Magnetfeld des Magnetkontakts 12 auf den Reedschalter 11 einwirkt. Dadurch wird der Reedschalter 11 geschaltet, wobei in der Sicherungsstellung die Energiespeichereinrichtung 2 mit dem Heizelement 4 getrennt ist. Befindet sich das Wärmekissen 1 wie hier dargestellt in der Betriebsstellung, so ist der Reedschalter 11 derart gestaltet, dass die Energiespeichereinrichtung 2 mit dem Heizelement 4 verbunden sein kann. Die zwischengeschaltete Steuereinrichtung 7 kann jedoch beispielsweise aufgrund der aktiven Ladeabschaltung und/oder Unterspannungsabschaltung die Verbindung zwischen Energiespeichereinrichtung 2 und Heizelement 4 weiterhin trennen.

Um das Wärmekissen 1 in der Sicherungsstellung zu halten, weist das Wärmekissen 1 Halteelemente 13 auf. Die Halteelemente 13 sind symmetrisch zur Faltebene FE angeordnet. Dadurch liegen die Halteelemente 13 in der Sicherungsstellung, wie in der Figur 4b dargestellt, aufeinander.

Das Wärmekissen 1 weist eine Hülle 14 auf, welche alle elektronischen Bauteile des Wärmekissens 1, insbesondere die Energiespeichereinrichtung 2, das Heizelement 4, die Leiterbahnen 6, die Steuereinrichtung 7, Ladeeinrichtung 8 und die Schalteinrichtung 10, ummantelt. Im dargestellten Ausführungsbeispiel ist die Hülle 14 geschnitten dargestellt, um die elektronischen Bauteile des Wärmekissens 1 einfacher darzustellen. Die Halteelemente 13 können ebenso in der Hülle 14 eingebettet und/oder innerhalb der Hülle 14 angeordnet sein. Die Hülle 14 kann aus einem flexiblen und nichtleitenden Material, insbesondere aus Kunststoff, vorzugsweise aus Silikon und/oder TPE, bestehen.

Die elektronischen Bauteile des Wärmekissens 1 werden im Herstellungsprozess vorteilhafterweise mit der Hülle 14 aus Kunststoff, insbesondere aus Silikon und/oder TPE, umspritzt oder umgossen. So können die Zwischenbereiche, zwischen den elektronischen Bauteilen des Wärmekissens 1, entgegen des hier dargestellten Ausführungsbeispiels, ebenso mit dem Material der Hülle 14 gefüllt werden. Aufgrund des flexiblen Materials der Hülle 14 kann das Wärmekissen 1 zudem sehr einfach entlang der Faltebene FE gefaltet werden.

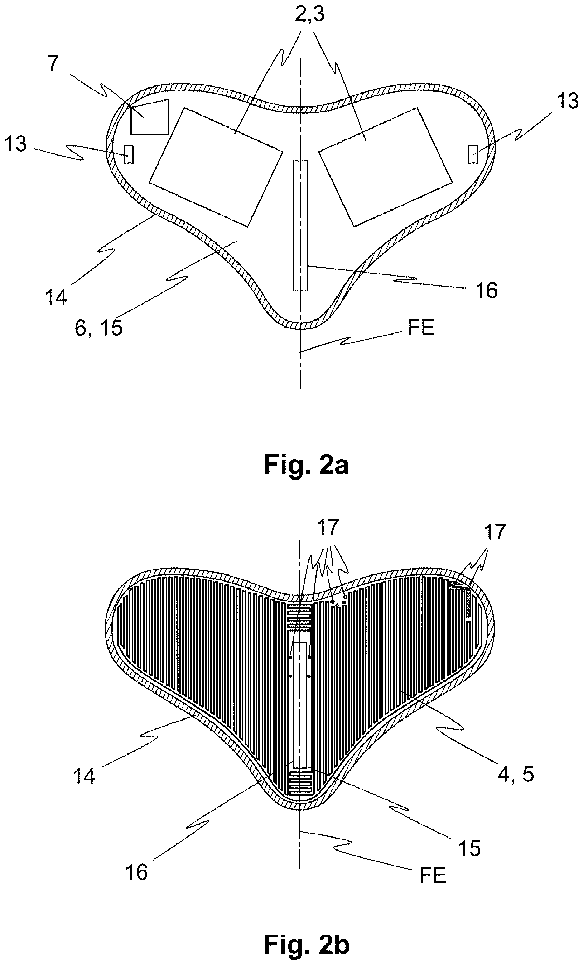

In Figur 2a ist eine schematische Draufsicht eines Wärmekissens 1 gemäß einem alternativen Ausführungsbeispiel dargestellt. Ebenso wie der Darstellung der Figur 1 ist auch hier die Hülle 14 geschnitten dargestellt, um die elektronischen Bauteile des Wärmekissens 1 vereinfacht darzustellen. Zusätzlich oder alternativ zum Ausführungsbeispiel der Figur 1 sind die Leiterbahnen 6 als Leiterplatte 15 ausgebildet. Die Leiterplatte 15 weist vorzugsweise eine Versorgungslage für die elektrische Energie und eine Signallage für die Signale auf. Zudem übernimmt die Leiterplatte 15 die Funktion, dass sie die elektronischen Bauteile des Wärmekissens 1, insbesondere die Energiespeichereinrichtung 2, die Steuereinrichtung 7 und die Schalteinrichtung 10, mechanisch und/oder elektrisch aufnimmt und miteinander verbindet. Zusätzlich kann die Leiterplatte 15 die Ladeeinrichtung 8 und/oder die Schalteinrichtung 10, wie in der Figur 1 dargestellt, aufnehmen.

Da die Leiterplatte 15 zumindest teilweise dem Wärmekissen 1 eine erhöhte Steifigkeit verleiht, weist die Leiterplatte 15 eine Aussparung 16 auf. Die Aussparung 16 ist dabei symmetrisch und entlang der Faltebene FE angeordnet. Durch die Aussparung 16 ist ein einfaches Falten des Wärmekissens 1 entlang der Faltebene FE realisierbar.

Figur 2b zeigt eine schematische Untersicht eines Wärmekissens 1 gemäß einem Ausführungsbeispiel ähnlich der Figur 2a. Ebenso wie die Darstellung der Figur 2a ist auch hier die Hülle 14 geschnitten dargestellt, um die elektronischen Bauteile des Wärmekissens 1 vereinfacht darzustellen. Im Gegensatz zur Figur 2a zeigt die Figur 2b ein ähnliches Ausführungsbeispiel in der Untersicht. Hier ist ein Heizelement 4 aus einem Heizdraht 5 dargestellt, welches sich im Wesentlichen über die gesamte Fläche des Wärmekissens 1 ohne Hülle 14 erstreckt. Das Heizelement 4 ist hierbei vorteilhafterweise als Heizlage an der Leiterplatte 15, insbesondere an der Unterseite der Leiterplatte 15, angeordnet. Das Heizelement 4 als Heizlage kann dabei über Kontaktstellen 17 mit der Versorgungslage und/oder der Signallage der Leiterplatte 15 verbunden sein. Ebenso ist es vorstellbar, dass das Heizelement 4 die Aussparung 16 aufweist.

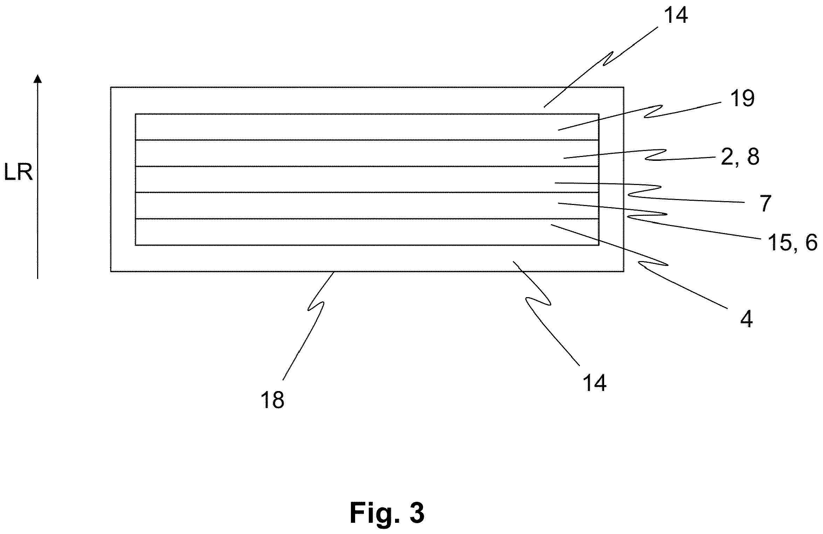

In Figur 3 ist ein schematischer Lagenaufbau eines Wärmekissens 1 gemäß einem weiteren Ausführungsbeispiel dargestellt. Hierbei ist lediglich ein Ausschnitt des Wärmekissens 1 dargestellt und zeigt somit lediglich den prinzipiellen Lageaufbau des Wärmekissens 1 gemäß dem weiteren Ausführungsbeispiel. Wie hier dargestellt, sind alle elektronischen Bauteile des Wärmekissens 1 im Inneren der Hülle 14 und somit, insbesondere vollständig, ummantelt. Der Bereich der Hülle 14, der am Körper anliegt, kann dabei als Anlagefläche 18 bezeichnet werden. Um die Wärme des Heizelements 4 in Richtung des Körpers und nicht an die Umgebung abzuleiten, ist das Heizelement 4 in eine Lagenaufbaurichtung LR, insbesondere unmittelbar, an der Hülle 14 im Bereich der Anlagefläche 18 angeordnet. Anschließend an das Heizelement 14 können die Leiterplatte 15 mit den Leiterbahnen 6, die Steuereinrichtung 7, die Energiespeichereinrichtung 2 und die Ladeeinrichtung 8 angeordnet sein. Diese können wie in Figur 2a und 2b dargestellt nebeneinander oder wie hier dargestellt, in Lagenaufbaurichtung LR übereinander angeordnet sein. Ebenso kann die Energiespeichereinrichtung 2 und die Ladeeinrichtung 8 in unterschiedlichen Lagen angeordnet sein. Um die Wärme nicht an die Umgebung abzugeben, weist das Wärmekissen 1 ein Isolationselement 19 auf. Das Isolationselement 19 ist im gezeigten Ausführungsbeispiel in Lagenaufbaurichtung LR maximal vom Heizelement 4 beabstandet. So wird die Wärme, die insbesondere von der Heizeinrichtung 4, der Steuereinrichtung 7, der Energiespeichereinrichtung 2 und/oder der Ladeeinrichtung 8 erzeugt wird, über die Anlagefläche 18 an den Körper abgegeben.

In den Figuren 4a und 4b ist jeweils eine schematische räumliche Darstellung des Wärmekissens 1 gemäß einem weiteren alternativen Ausführungsbeispiel dargestellt. Für eine übersichtlichere Darstellung wurde hier im Gegensatz zu den vorangegangenen Figuren auf die Darstellung der elektronischen Bauteile im Inneren des Wärmekissens 1, insbesondere die Energiespeichereinrichtung 2, das Heizelement 4, die Leiterbahnen 6, die Steuereinrichtung 7, Ladeeinrichtung 8 und/oder die Schalteinrichtung 10, verzichtet.

Die Figur 4a zeigt das Wärmekissen 1 in einer Betriebsstellung. Die Figur 4b zeigt das Wärmekissen 1 in einer Sicherungsstellung. Das Wärmekissen 1 wird entlang einer Faltkontur 20, welche sich im Wesentlichen mittig an der Faltebene FE der Figuren 1-3 erstreckt, gefaltet. Mittels der Halteelemente 12 kann das Wärmekissen 1 in der Sicherungsstellung, wie in der Figur 4b dargestellt, gehalten werden. Die Faltkontur 20 ist im gezeigten Ausführungsbeispiel an der Anlagefläche 18 ausgebildet. Wird das Wärmekissen 1 in die Sicherungsstellung gefaltet, so befindet sich die Anlagefläche 18 auf der Innenseite des gefalteten Wärmekissens 1. Ist der Lagenaufbau des Wärmekissens 1 ähnlich zu dem in der Figur 3 dargestellten Lagenaufbaus ausgebildet, so befindet sich in der Sicherungsstellung das Heizelement 4 näher an der Innenseite und die Ladeeinrichtung 8 näher an der Außenseite des Wärmekissens 1.

Die vorliegende Erfindung ist nicht auf die dargestellten und beschriebenen Ausführungsbeispiele beschränkt. Abwandlungen im Rahmen der Patentansprüche sind ebenso möglich wie eine Kombination der Merkmale, auch wenn diese in unterschiedlichen Ausführungsbeispielen dargestellt und beschrieben sind.

### Bezugszeichenliste

- 1: Wärmekissen
- 2: Energiespeichereinrichtung
- 3: Akkumulator
- 4: Heizelement
- 5: Widerstandsdraht
- 6: Leiterbahn
- 7: Steuereinrichtung
- 8: Ladeeinrichtung
- 9: Induktionsspule
- 10: Schalteinrichtung
- 11: Reedschalter
- 12: Magnetkontakt
- 13: Halteelemente
- 14: Hülle
- 15: Leiterplatte
- 16: Aussparung
- 17: Kontaktstellen
- 18: Anlagefläche
- 19: Isolationselement
- 20: Faltkontur
- FE: Faltebene

## Patentansprüche

1. Wärmekissen (1) für den menschlichen Körper zur Linderung von körperlichen Beschwerden, insbesondere Dysmenorrhoe,
mit wenigstens einer Energiespeichereinrichtung (2) zum Speichern von elektrischer Energie und
einem Heizelement (4) zum Erwärmen des Wärmekissens (1) mittels der elektrischen Energie,
**dadurch gekennzeichnet, dass**
das Wärmekissen (1) wenigstens eine Leiterbahn (6) zum Leiten von Signalen und/oder der elektrischen Energie aufweist,
wobei das Heizelement (4), die Energiespeichereinrichtung (2) und die Leiterbahn (6), insbesondere vollständig, von einer Hülle (14) ummantelt sind.

2. Wärmekissen (1) nach dem vorangegangenen Anspruch, **dadurch gekennzeichnet, dass** das Wärmekissen (1) wenigstens eine Leiterplatte (15) aufweist, wobei die wenigstens eine Leiterbahn (6) als Versorgungslage für die elektrische Energie und/oder als Signallage für die Signale an der Leiterplatte (15) angeordnet ist.

3. Wärmekissen (1) nach einem der vorangegangenen Ansprüche,
**dadurch gekennzeichnet, dass** das Heizelement (4) als Heizlage an der Leiterplatte (15) angeordnet ist und/oder unmittelbar an der Hülle (14) anliegt.

4. Wärmekissen (1) nach einem der vorangegangenen Ansprüche,
**dadurch gekennzeichnet, dass** das Wärmekissen (1) eine Ladeeinrichtung (8), zum Laden der Energiespeichereinrichtung (2) aufweist.

5. Wärmekissen (1) nach einem der vorangegangenen Ansprüche, **dadurch gekennzeichnet, dass** die Ladeeinrichtung (8) eine Induktionsspule (9) aufweist, wobei die Ladeeinrichtung (8), insbesondere vollständig, von der Hülle (14) ummantelt ist.

6. Wärmekissen (1) nach einem der vorangegangenen Ansprüche, **dadurch gekennzeichnet, dass** das Wärmekissen (1) ein Isolationselement (19) zur thermischen Isolation der Ladeeinrichtung (8), der Energiespeichereinrichtung (2) und/oder der Leiterplatte (15) zur Hülle (14) und/oder zum Heizelement (4) aufweist.

7. Wärmekissen (1) nach einem der vorangegangenen Ansprüche, **dadurch gekennzeichnet, dass** die Energiespeichereinrichtung (2) eine Vielzahl an Akkumulatoren (3) aufweist und/oder die Energiespeichereinrichtung (2) zumindest teilweise flexibel ausgestaltet ist.

8. Wärmekissen (1) nach einem der vorangegangenen Ansprüche, **dadurch gekennzeichnet, dass** die Leiterplatte (15) wenigstens eine Aussparung (16) aufweist und/oder zumindest teilweise aus einem flexiblen Material besteht.

9. Wärmekissen (1) nach einem der vorangegangenen Ansprüche, **dadurch gekennzeichnet, dass** die Leiterplatte (15), die Hülle (14), das Heizelement (4), die Energiespeichereinrichtung (2) und/oder die Aussparung (16) zu einer Faltebene (FE) symmetrisch zum Falten des Wärmekissens (1) von einer Betriebsstellung in eine Sicherungsstellung und zurück ausgebildet ist.

10. Wärmekissen (1) nach einem der vorangegangenen Ansprüche, **dadurch gekennzeichnet, dass** das Wärmekissen (1) wenigstens zwei Halteelemente (13) zum Halten des Wärmekissens (1) in der Sicherungsstellung aufweist, wobei die Halteelemente (13) vorzugsweise symmetrisch zur Faltebene (FE) angeordnet sind.

11. Wärmekissen (1) nach einem der vorangegangenen Ansprüche, **dadurch gekennzeichnet, dass** das Wärmekissen (1) eine Schalteinrichtung (10) zum Schalten des Wärmekissens (1) aufweist, wobei die Schalteinrichtung (10) vorzugsweise wenigstens einen Reedschalter (11) und/oder einen Magnetkontakt (12) aufweist.

12. Wärmekissen (1) nach einem der vorangegangenen Ansprüche, **dadurch gekennzeichnet, dass** die Schalteinrichtung (10), insbesondere der Reedschalter (11) und/oder der Magnetkontakt (12), derart eingerichtet ist, dass das Heizelement (4) in der Sicherungsstellung von der Energiespeichereinrichtung (2) unterbrochen ist.

13. Wärmekissen (1) nach einem der vorangegangenen Ansprüche, **dadurch gekennzeichnet, dass** die Schalteinrichtung (10), insbesondere der Reedschalter (11) und/oder der Magnetkontakt (12), derart eingerichtet ist, dass das Heizelement (4) in der Betriebsstellung mit der Energiespeichereinrichtung (2) verbunden ist.

14. Wärmekissen (1) nach einem der vorangegangenen Ansprüche, **dadurch gekennzeichnet, dass** das Wärmekissen (1) eine Steuereinrichtung (7) zum Steuern und/oder Regeln der Energiespeichereinrichtung (2), des Heizelements (4), der Ladeeinrichtung (8) und/oder der Schalteinrichtung (10) aufweist, wobei die Steuereinrichtung (7) vorzugsweise mit der Signallage der Leiterplatte (15) zur Verarbeitung der Signale verbunden ist.

15. Wärmekissen (1) nach einem der vorangegangenen Ansprüche, **dadurch gekennzeichnet, dass** die Energiespeichereinrichtung (2), das Heizelement (4), die Steuereinrichtung (7), die Schalteinrichtung (10) und/oder die Ladeeinrichtung (8) elektrisch leitend mit der wenigstens einen Leiterbahn (6), vorzugsweise mit der Leiterplatte (15), insbesondere der Signallage und/oder der Versorgungslage der Leiterplatte (15), verbunden ist.
